# EUROPÄISCHE PATENTSCHRIFT

(11) **EP 0 317 744 B1**
(45) Veröffentlichungstag und Bekanntmachung des Hinweises auf die Patenterteilung: **24.11.1993**
(21) Anmeldenummer: 88116319.0
(22) Anmeldetag: 03.10.1988
(51) Int. Cl.: C07D 229/00, C08G 18/79

(54) **Verfahren zur Herstellung (cyclo)-aliphatischer Uretdione**
Process for the preparation of (cyclo)-aliphatic uret diones
Procédé de préparation d'uretdiones(cyclo)-aliphatiques

(30) Priorität: 21.11.1987 DE 3739549
(43) Veröffentlichungstag der Anmeldung: 31.05.1989
(73) Patentinhaber: HÜLS AKTIENGESELLSCHAFT, 45764 Marl (DE)
(72) Erfinder: Disteldorf, Josef, Dr., D-4370 Marl (DE); Hübel, Werner, Dr., D-4350 Recklinghausen (DE); Schmitz, Karl, Dr., D-4390 Gladbeck (DE)

(56) Entgegenhaltungen:
- EP-A- 0 045 995
- DE-A- 1 694 485
- DE-A- 2 452 390
- DE-B- 1 081 895
- DE-B- 1 230 425
- FR-A- 2 298 566
- US-A- 2 643 250
- US-A- 2 683 144
- CHEMICAL ABSTRACTS, Band 87, Nr. 17, 24. Oktober 1978, Seite 724, Spalte 2, Zusammenfassung Nr. 135294r, Columbus, Ohio, US

## Beschreibung

Gegenstand der Erfindung ist ein verbessertes Verfahren zur Herstellung weitgehend isocyanuratfreier Uretdione aus (cyclo)aliphatischen Diisocyanaten. Derartige Uretdione lassen sich zu lichtechten Ein- und Zweikomponenten-Polyurethanlacken verarbeiten. Für eine Reihe von Anwendungen ist die Anwesenheit von Isocyanuraten unerwünscht, da diese bekanntlich trifunktionell sind und zu Vernetzungen neigen. In der Praxis wird bereits eine Beimengung von mehr als 0,5 % als störend empfunden.

Es ist grundsätzlich bekannt, Uretdione durch Dimerisierung von Isocyanaten in Gegenwart bestimmter Katalysatoren herzustellen. In der Vergangenheit sind zu diesem Zweck Antimonpentafluorid, Trialkylphosphine, aminosubstituierte Phosphine, Imidazole, Guanidine und Pyridine vorgeschlagen worden.

Der Nachteil der Anwendung von Antimonpentafluorid (vgl. DE-OS 34 20 114) besteht darin, daß diese korrosive und teure Verbindung vor der destillativen Aufarbeitung mit der fünffachen Menge Zinkpulver zerstört und der Niederschlag aus Antimon und Zinkfluorid abfiltriert werden muß.

Aus der FR-PS 15 32 054 ist ein Verfahren bekannt geworden, bei dem man als Dimerisierungskatalysatoren tert. Phosphine oder Bortrifluorid einsetzt. Diese katalysieren jedoch nicht nur die Dimerisierung, sondern in beachtlichem Maße auch die Trimerisierung von Isocyanaten. Bortrifluorid ist darüber hinaus wegen seiner großen Korrosivität nur unter besonderen Schutzmaßnahmen einsetzbar.

1,2-Dimethylimidazol ist ein ausgezeichneter Dimerisierungskatalysator für aromatische Isocyanate (vgl. Synthesis 1975, Seite 463 ff.). Bei Isocyanaten, die keine aromatischen NCO-Gruppen aufweisen, ist dieser Katalysator jedoch deutlich weniger selektiv. Beispielsweise erhält man beim Einsatz von Benzylisocyanat ein Gemisch aus 24 % Isocyanurat und nur 76 % Uretdion.

In der Praxis haben sich als Dimerisierungskatalysatoren aminosubstituierte Phosphine durchgesetzt.

Nach dem Verfahren der DE-OS 30 30 513 stellt man das Uretdion des Isophorondiisocyanats durch Dimerisierung des Monomeren in Gegenwart eines organischen Phosphor-Stickstoff-Katalysators und anschließende destillative Aufarbeitung im Dünnschichtverdampfer her. Die Uretdione verbleiben im Destillationsrückstand, während das nicht umgesetzte Monomere mit der Hauptmenge des eingesetzten Katalysators als Destillat anfällt, das in den Prozeß zurückgeführt wird. Als bevorzugter Katalysator wird Tris-(N,N-dimethylamino)-phosphin (PTD) genannt. Uretdione, die in Gegenwart von PTD hergestellt wurden, enthalten typischerweise 1 % Isocyanurat, 1 % Monomere und 0,01 bis 0,1 % Katalysator.

In der DE-OS 34 37 635 wird vorgeschlagen, zur Dimerisierung organischer Isocyanate Katalysatoren des gleichen Typs unter Mitverwendung von H-aktiven organischen Verbindungen wie Alkoholen, Phenolen, (cyclo)aliphatischen Aminen, Amiden, Urethanen und Harnstoffen zu verwenden. Besonders bevorzugte Katalysatoren sind PTD und Tris-(N,N-diethylamino)-phosphin. Werden di- oder höherfunktionelle Isocyanate eingesetzt, wird die Reaktion üblicherweise bei Erreichen eines Dimerisierungsgrades von 10 - 50 % durch Zugabe eines Katalysatorgiftes, wie z. B. Chloressigsäure, abgebrochen (vgl. Seite 16, 1. Absatz). Ein Problem stellt die Isolierung des Uretdions dar. Unter den Bedingungen der Dünnschichtdestillation besteht nämlich die Gefahr einer katalysierten Rückspaltung der vorliegenden Uretdione in die Ausgangsisocyanate (vgl. Seite 16 Mitte). Falls sich der Katalysator mit dem überschüssigen Isocyanat abdestillieren läßt, kann auf eine Deaktivierung des Katalysators verzichtet werden. Jedoch können dann während und nach der Aufarbeitung unkontrollierbare Nebenreaktionen ablaufen (vgl. Seite 20).

Es ist bekannt, daß PTD dazu neigt, in Gegenwart von Luftsauerstoff zu Hexamethylphoshorsäuretriamid zu reagieren, das bekanntlich im Verdacht steht, cancerogen zu sein (vgl. Br. J. Cancer 38, 418 - 427 (1978). Deshalb sollte auf den Einsatz von PTD verzichtet werden. Die genannten Verfahren weisen darüber hinaus den Nachteil auf, daß die Katalysatoren Nebenreaktionen eingehen und so zum Teil verbraucht werden. Beim erstgenannten Verfahren müssen daher die Katalysatorverluste regelmäßig ausgeglichen werden. Beim letztgenannten Verfahren verbleibt der eingesetzte Katalysator nach der Deaktivierung zu einem erheblichen Anteil im Uretdion. Beide Verfahren werden daher durch hohe Katalysatorkosten belastet.

Aus der Literatur sind auch weitere Dimerisierungskatalysatoren bekannt. So wird z. B. in der JP-AS 71/37 503 die Dimerisierung von 2,4-Tolylendiisocyanat mit cylischen Amidinen, wie 1,8-Diazabicyclo[5.4.0]undec-7-en, beschrieben. Versuche der Anmelderin zeigen, daß man mit diesem Katalysator (cyclo)aliphatische Diisocyanate nicht dimerisieren kann (siehe Vergleichsbeispiel A). Offensichtlich ist die bekannterweise geringere Reaktivität dieser Diisocyanate nicht ausreichend, um eine Reaktion zu ermöglichen.

Gegenstand der DE-PS 10 81 895 (entspricht US-PS 3 144 452) ist ein Verfahren zur Herstellung von N,N-Diaryluretdionen und Triarylisocyanursäureestern durch Di- bzw. Trimerisierung von aromatischen Isocyanaten. Als Katalysatoren werden in 3- oder 4-Stellung substituierte Pyridine mit einer bestimmten Basizität eingesetzt. Unter anderem werden durch Alkylgruppen substituierte 4-Aminopyridine erwähnt. Nach diesem Verfahren soll es möglich sein, je nach Menge des Katalysators, Reaktionstemperatur und Art des verwendeten Lösemittels Uretdione, Isocyanurate oder deren Mischungen zu erhalten. So wird beispielsweise empfohlen, zur Herstellung von Uretdionen den Katalysator in einer Menge von 0,005 bis 15 %, bezogen auf Gewicht des eingesetzten Isocyanats, einzusetzen, bei möglichst niedriger Temperatur zu arbeiten und ein inertes organisches Lösemittel zu verwenden, in dem das Uretdion schlecht löslich ist.

Die Tatsache, daß die für die Herstellung von Isocyanuraten empfohlene Katalysatormenge sich mit der vorstehend angegebenen in weiten Bereichen überschneidet und auch die beiden anderen Reaktionsparameter nicht klar abgrenzbar sind, gibt dem Fachmann Anlaß, an der Möglichkeit der selektiven Reaktionssteuerung zu zweifeln. Vergleichsversuche der Anmelderin zeigen in der Tat, daß stets die beiden Oligomeren gebildet werden. Bei der Nacharbeitung von Beispiel 2 wurden z. B. 2 Gewichtsprozent Isocyanurat erhalten. Wird das Reaktionsgemisch kurzfristig auf 145 °C erhitzt, erhält man sogar 10 Gewichtsprozent Isocyanurat, während gleichzeitig das Uretdion teilweise in das Monomere gespalten wird.

Aus einer späteren Anmeldung des Patentinhabers geht hervor, daß 4,4'-Diphenylmethan-diisocyanat in Gegenwart von 4-Dimethylaminopyridin bei Raumtemperatur zu trimeren und höher oligomeren Produkten umgesetzt wird (vgl. DE-AS 16 94 485). Auch hieraus ist abzuleiten, daß die Pyridinderivate offenbar stets beide Reaktionen, die Dimerisierung und die Trimerisierung, katalysieren. Zur Herstellung von Uretdionen, die praktisch frei von Isocyanuraten sein sollen, erscheinen die Pyridinderivate daher nicht geeignet. Dies gilt insbesondere für aliphatische Isocyanate, da diese im Gegensatz zu den aromatischen Isocyanaten entweder keine Uretdione bilden oder nur unter Einhaltung spezieller Reaktionsbedingungen in der Lage sind, die dimeren Additionsprodukte zu bilden (vgl. J. Org. Chem. 36, 3056 (1971).

Während also zahlreiche Verfahren zur Dimerisierung von aromatischen Diisocyanaten bekannt sind, gibt es praktisch nur eine Möglichkeit, (cyclo)aliphatische Diisocyanate zu dimerisieren, und diese beruht auf der Verwendung der unerwünschten Aminophosphine (siehe DE-OS 34 37 635).

Das in der JP-OS 84/98 180 beschriebene Verfahren zur Oligomerisierung von (cyclo)aliphatischen Diisocyanaten ist für die gezielte Herstellung von Uretdionen nicht geeignet, da bekannt ist, daß die erhaltenen Gemische aus Uretdionen und Isocyanuraten nur unter großen Schwierigkeiten trennbar sind. Uretdione neigen dazu, in der Wärme in ihre Ausgangsprodukte zurückzuspalten. Hiermit ist insbesondere dann zu rechnen, wenn noch Katalysatorreste zugegen sind.

Ziel der vorliegenden Erfindung war es somit, ein Verfahren zur katalytischen Herstellung von (cyclo)aliphatischen Uretdionen mit einem Reinheitsgrad von mehr als 99 % bereitzustellen, das nicht auf die Verwendung der teuren und potentiell cancerogenen Aminophosphine angewiesen ist.

Es wurde jetzt überraschend ein solches Verfahren gefunden. Es besteht darin, daß man ein aliphatisches und/oder cycloaliphatisches Diisocyanat mit jeweils 6 bis 15 C-Atomen in Gegenwart eines Pyridins der allgemeinen Formel 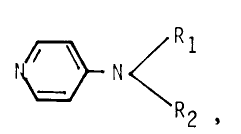
wobei R₁ und R₂ jeweils unabhängig voneinander für einen Alkylrest mit 1 bis 4 C-Atomen stehen oder zusammen mit dem Stickstoff einen Pyrrolidin-, Piperidin- oder Morpholinring bilden können, dimerisiert und das Reaktionsgemisch nach Erreichen eines Dimerisierungsgrades von 10 bis 80 %, insbesondere 20 bis 60 %, einer Vakuumdünnschichtverdampfung zur Isolierung des Uretdions unterwirft. Es ist also nicht erforderlich, die Reaktion durch Zugabe eines Katalysatorgiftes abzustoppen. Als Diisocyanat wird Isophorondiisocyanat bevorzugt. Das substituierte Pyridin, insbesondere p-Dimethylaminopyridin, wird in einer Menge von 0,05 bis 10 Gewichtsprozent, vorzugsweise von 0,2 bis 5 Gew.-%, eingesetzt. Die Dimerisierung wird vorzugsweise bei einer Temperatur von 0 bis 100 °C, die Dünnschichtverdampfung je nach dem angelegten Vakuum im Bereich von 0,1 bis 20 mbar bei einer Temperatur von 150 bis 190 °C vorgenommen. Das Destillat der Dünnschichtverdampfung kann wieder zurückgeführt werden. Es empfiehlt sich, die Dimerisierung in Gegenwart eines Schutzgases durchzuführen.

Es sind also von den in der DE-PS 10 81 895 erwähnten substituierten Pyridinen lediglich die 4-Dialkylaminopyridine als Dimerisierungskatalysatoren geeignet. Es ist auf Grund des Standes der Technik überraschend, daß diese Pyridine hochselektive Katalysatoren für die Dimerisierung von (cyclo)aliphatischen Diisocyanaten sind.

Gegenstand der Erfindung ist ein Verfahren zur Herstellung von Uretdionen mit einem Reinheitsgrad von mehr als 99 % durch Umsetzung der entsprechenden Diisocyanate unter wasserfreien Bedingungen mit Pyridinen der allgemeinen Formel 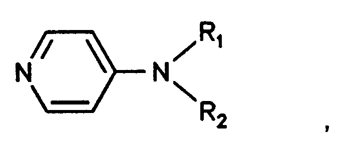
wobei R₁ und R₂ jeweils unabhängig von einander für einen Alkylrest mit 1 bis 4 C-Atomen stehen oder zusammen mit dem Stickstoff einen Pyrrolidin-, Piperidin- oder Morpholinring bilden können, und anschließende Aufarbeitung des Reaktionsgemisches,
welches dadurch gekennzeichnet ist,
daß man
- als Diisocyanat ein (cyclo)aliphatisches Diisocyanat oder ein entsprechendes Diisocyanatgemisch mit jeweils 6 bis 15 C-Atomen einsetzt,
- die Reaktionstemperatur bei 0 bis 100 °C liegt und
- das Reaktionsgemisch nach Erreichen eines Dimerisierungsgrades von 10 bis 80 %, insbesondere 20 bis 60 %, einer Vakuumdünnschichtverdampfung bei einem Vakuum von 0,1 bis 20 mbar und einer Temperatur von 150 - 180 °C zur Isolierung des Uretdions unterwirft.

Erstaunlich ist es, daß man nach diesem Verfahren Uretdione in hoher Reinheit erhält. Aufgrund des Standes der Technik hätte man erwartet, daß bereits während der Reaktion zu einem bestimmten Anteil Isocyanurate gebildet werden. Die Vakuumdünnschichtverdampfung erfordert kurzzeitig bei Temperaturen von 150 bis maximal 190 °C bei einem Vakuum im Bereich von 0,1 bis 20 mbar. Selbst unter diesen für Uretdione drastischen Temperaturbedingungen kommt es nicht zu einer nennenswerten Bildung von Isocyanuraten. Insgesamt liegt der Anteil des gebildeten Isocyanurats bei dem erfindungsgemäßen Verfahren unter 0,5 %, bezogen auf die zur gleichen Zeit hergestellte Gewichtsmenge an Uretdion.

Vorteilhaft ist auch, daß die als Katalysator benötigten substituierten Pyridine leicht zugängliche Handelsprodukte sind.

Schließlich läßt sich der Reaktionsverlauf bei Verwendung der substituierten Pyridine wesentlich besser steuern, da nahezu keine Nebenreaktionen stattfinden.

Im folgenden soll das erfindungsgemäße Verfahren näher beschrieben werden.

Als Diisocyanate eignen sich aliphatische und cycloaliphatische Diisocyanate mit 6 bis 15 C-Atomen, vorzugsweise 8 bis 12 C-Atomen, wie z. B. Hexamethylendiisocyanat, Dodekamethylendiisocyanat und Bis-(4-isocyanatocyclohexyl)-methan sowie deren Gemische. Bevorzugte aliphatische Diisocyanate sind 2-Methylpentamethylendiisocyanat, sowie 2,2,4- und 2,4,4-Trimethylhexamethylendiisocyanat. Unter den cycloaliphatischen Diisocyanaten wird Isophorondiisocyanat bevorzugt.

Das als Dimerisierungskatalysator verwendete Pyridin weist die allgemeine Formel 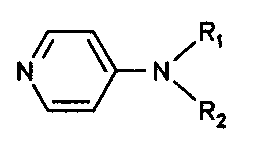
auf. R₁ und R₂ haben unabhängig voneinander die Bedeutung eines Alkylrestes mit 1 bis 4 C-Atomen oder bilden zusammen mit dem benachbarten Stickstoffatom einen Pyrrolidin-, Piperidin- oder Morpholinring. Im Vergleichsversuch (siehe Tabellen 4 und 5) werden die besten Ergebnisse mit p-Pyrrolidinopyridin erzielt. Aufgrund ihrer besseren Verfügbarkeit bevorzugt man jedoch als Katalysator 4-Dimethylaminopyridin und/oder 4-Diethylaminopyridin. Der Katalysator wird in einer Menge von 0,05 bis 10 %, insbesondere 0,2 bis 5 %, bezogen auf die Gewichtsmenge des eingesetzten Diisocyanats, eingesetzt.

Die Dimerisierung kann in Gegenwart von Lösemitteln durchgeführt werden, die gegenüber Diisocyanaten inert sind. Allerdings bringt eine solche Verfahrensvariante in der Regel keine besonderen Vorteile mit sich. Geeignet sind insbesondere Hexan, Toluol, Xylol, Chlorbenzol und deren Gemische.

Die Reaktionstemperatur liegt üblicherweise zwischen 0 und 100 °C, vorzugsweise zwischen 20 und 80 °C. Die Dimerisierung wird vorteilhafterweise in Gegenwart eines Schutzgases, wie z. B. Stickstoff, durchgeführt. Man arbeitet üblicherweise bei Normaldruck. Die Reaktionszeiten betragen im allgemeinen 1 bis 5 Tage. Sie hängen hauptsächlich von der Katalysatorkonzentration ab.

Sobald ein Dimerisierungsgrad von 10 bis 80 %, vorzugsweise von 20 bis 60 %, erreicht ist, wird der Reaktionsansatz direkt einer Vakuumdünnschichtverdampfung unterworfen. Es wird also vorteilhafterweise auf eine Desaktivierung des Katalysators verzichtet. Bei der Dünnschichtverdampfung zwecks IPDI-Uretdion-Herstellung stellt man im Vorverdampfer insbesondere eine Temperatur von 180 °C und einen Druck von 0,55 mbar ein. Im Hauptverdampfer beträgt die Temperatur insbesondere 165 °C und der Druck 0,05 mbar. Die Verweilzeit in Vorverdampfer und Hauptverdampfer beträgt dann jeweils ca. 1 Minute.

Bei der Vakuumdünnschichtverdampfung fällt als Destillationsrückstand das Uretdion in einer Reinheit von mehr als 99 % an. Der Gehalt an Monomeren wird gaschromatographisch bestimmt. Der Anteil des Isocyanurats im Endprodukt liegt unter 0,5 %, der Monomergehalt unter 0,4 %; Katalysatorreste sind nicht nachweisbar.

Das Destillat besteht aus monomerem Diisocyanat sowie Katalysator. Es kann vorteilhafterweise wieder dem Dimerisierungsprozeß zugeführt werden.

Die Bestimmung des Dimerisierungsgrades und die Beobachtung des Reaktionsverlaufes erfolgen mit Hilfe der NCO-Zahl. Die NCO-Zahl wird nach der Methode bestimmt, die in Houben-Weyl "Methoden der organischen Chemie" Band 14/2, Stuttgart (1963) S. 85 angegeben ist.

Der Gehalt an Isocyanurat im Reaktionsprodukt wird qualitativ mit Hilfe des IR-Spektrums und quantitativ durch Bestimmung des NCO-Heißwertes ermittelt. Zur Bestimmung des NCO-Heißwertes wird die Probe 2 Stunden in Dichlorbenzol gekocht. Anschließend wird die übliche Bestimmung der NCO-Zahl vorgenommen.

### Beispiel 1

Eine 100 g-Probe eines (cyclo)-aliphatischen Diisocyanats, die 1 Gew.-% p-Dimethylamino-pyridin enthält, wird unter Stickstoff 24 Stunden auf 70 °C gehalten. Anschließend ermittel man den Dimerisierungsgrad. Die gefundenen Werte sind Tabelle 1 zu entnehmen.

**Tabelle 1**

| (cyclo)aliphatisches Diisocyanat | Dimerisierungsgrad |
|---|---|
| 2-Methylpentan-1,5-diisocyanat | 33,8 % |
| Isomerengemisch aus 2,2,4- und 2,4,4-Trimethylhexamethylendiisocyanat | 25,6 % |
| Hexan-1,6-diisocyanat | 29,9 % |
| Isophorondiisocyanat | 26,6 % |

### Beispiel 2

100 g-Proben Isophorondiisocyanat, die unterschiedliche Gewichtsmengen p-Dimethylaminopyridin enthalten, werden unter Stickstoff 1 Tag bzw. 5 Tage bei Raumtemperatur stehengelassen. Anschließend ermittelt man den in Tabelle 2 aufgeführten Dimerisierungsgrad.

**Tabelle 2**

| Beispiel | Gewichtsanteil p-Dimethylaminopyridin in % | Dimerisierungsgrad | |
|---|---|---|---|
| | | nach 1 Tag | nach 5 Tagen |
| 2.1 | 0,5 | 11,1 % | 33,3 % |
| 2.2 | 1,0 | 28,6 % | 49,2 % |
| 2.3 | 2,0 | 33,9 % | 61,9 % |
| 2.4 | 5,0 | 55,0 % | 74,1 % |

### Beispiel 3

Isophorondiisocyanatproben, die unterschiedliche Gewichtsanteile p-Dimethylaminopyridin enthalten, werden unter Stickstoff bei Raumtemperatur stehen gelassen. Anschließend erfolgt die destillative Aufarbeitung im Vakuumdünnschichtverdampfer. Die dabei erzielten Versuchsergebnisse sind Tabelle 3 zu entnehmen.

**Tabelle 3**

| | Beispiel 3.1 | Beispiel 3.2 | Beispiel 3.3 |
|---|---|---|---|
| Isophorondiisocyanat-Ansatz (kg) | 2,97 | 2,94 | 2,85 |
| p-Dimethylaminopyridin (Gew.-%) | 1,0 | 2,0 | 5,0 |
| Reaktionszeit (Stunden) | 120 | 67 | 17,5 |
| Destillat Gew.-% des Ansatzes | 57,3 | 58,2 | 63,1 |
| Uretdion-Ausbeute Gew.-% des Ansatzes | 42,7 | 41,8 | 36,9 |

### Beispiel 4

Eine 100 g-Isophorondiisocyanat-Probe, die 5 Gew.-% Dimerisierungskatalysator enthält, wird unter Stickstoff 1 Tag auf Raumtemperatur gehalten. Anschließend bestimmt man den Dimerisierungsgrad, der in Tabelle 4 aufgeführt ist.

**Tabelle 4**

| Beispiel | Katalysator | Dimerisierungsgrad |
|---|---|---|
| 4.1 | p-Pyrrolidinopyridin | 61,4 % |
| 4.2 | p-Diethylaminopyridin | 55,0 % |
| 4.3 | p-Piperidinopyridin | 50,3 % |
| 4.4 | p-Morpholinopyridin | 16,9 % |

### Beispiel 5

Eine 100 g Isophorondiisocyanat-Probe, die 1 Gew.-% Dimerisierungskatalystor enthält, wird unter Stickstoff 2 Tage auf Raumtemperatur gehalten. Anschließend ermittelt man den in Tabelle 5 angegebenen Dimerisierungsgrad.

**Tabelle 5**

| Beispiel | Katalysator | Dimerisierungsgrad nach 2 Tagen |
|---|---|---|
| 5.1 | p-Pyrrolidinopyridin | 48,1 |
| 5.2 | p-Diethylaminopyridin | 48,1 |
| 5.3 | p-Dimethylaminopyridin | 39,7 |
| 5.4 | p-Piperidinopyridin | 30,2 |

### Vergleichsbeispiele A bis D

Eine 100 g Isophorondiisocyanat-Probe, die 1 Gewichtsprozent Dimerisierungskatalysator enthält, wird unter Stickstoff 1 Tag bzw. 10 Tage auf Raumtemperatur gehalten. Anschließend ermittelt man den in Tabelle 6 angegebenen Dimerisierungsgrad.

**Tabelle 6**

| Vergleichsbeispiel | Katalysator | Dimerisierungsgrad nach | |
|---|---|---|---|
| | | 1 Tag | 10 Tagen |
| A | 1,8-Diazabicyclo[5.4.0]undec-7-en | 0 | 0 |
| B | 1,2-Dimethylimidazol | 0 | 0 |
| C | 3-Dimethylaminopyridin | 0 | 0 |
| D | 2-Dimethylaminopyridin | 0 | 0 |

## Patentansprüche

1. Verfahren zur Herstellung von Uretdionen mit einem Reinheitsgrad von mehr als 99 % durch Umsetzung der entsprechenden Diisocyanate unter wasserfreien Bedingungen mit Pyridinen der allgemeinen Formel 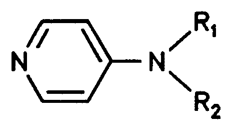 wobei R₁ und R₂ jeweils unabhängig von einander für einen Alkylrest mit 1 bis 4 C-Atomen stehen oder zusammen mit dem Stickstoff einen Pyrrolidin-, Piperidin- oder Morpholinring bilden können, und anschließende Aufarbeitung des Reaktionsgemisches,
dadurch gekennzeichnet,
daß man
- als Diisocyanat ein (cyclo)aliphatisches Diisocyanat oder ein entsprechendes Diisocyanatgemisch mit jeweils 6 bis 15 C-Atomen einsetzt,
- die Reaktionstemperatur bei 0 bis 100 °C liegt und
- das Reaktionsgemisch nach Erreichen eines Dimerisierungsgrades von 10 bis 80 %, insbesondere 20 bis 60 %, einer Vakuumdünnschichtverdampfung bei einem Vakuum von 0,1 bis 20 mbar und einer Temperatur von 150 - 190 °C zur Isolierung des Uretdions unterwirft.

2. Verfahren gemäß Anspruch 1,
dadurch gekennzeichnet,
daß man als Diisocyanat Isophorondiisocyanat einsetzt.

3. Verfahren gemäß den Ansprüchen 1 und 2,
dadurch gekennzeichnet,
daß man das substituierte Pyridin in einer Menge von 0,05 bis 10 %, insbesondere 0,2 bis 5 %, bezogen auf das Gewicht des eingesetzten Diisocyanats, einsetzt.

4. Verfahren gemäß den Ansprüchen 1 bis 3,
dadurch gekennzeichnet,
daß die Substituenten R₁ und R₂ des Pyridins für eine Methyl- oder Ethylgruppe stehen.

5. Verfahren gemäß den Ansprüchen 1 bis 4,
dadurch gekennzeichnet,
daß die Dimerisierung bei einer Temperatur von 20 bis 80 °C durchgeführt wird.

6. Verfahren gemäß den Ansprüchen 1 bis 4,
dadurch gekennzeichnet,
daß man die Dimerisierung in Gegenwart von Lösemitteln durchführt.

7. Verfahren gemäß den Ansprüchen 1 bis 6,
dadurch gekennzeichnet,
daß das im Vakuumdünnschichtverdampfer anfallende Destillat, das aus unumgesetzem Diisocyanat und Katalysator besteht, wieder zuzurückgeführt wird.

8. Verfahren gemäß den Ansprüchen 1 bis 6,
dadurch gekennzeichnet,
daß man die Dimerisierung in Anwesenheit eines Schutzgases durch führt.

## Claims

1. A process for the manufacture of urethane diones having a greater than 99% degree of purity by the reaction of the corresponding diisocyanates under anhydrous conditions with pyridines having the general formula 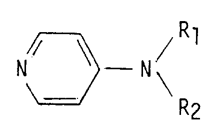 wherein R₁ and R₂ represent independent alkyl residues comprising 1 to 4 carbon atoms or together with the nitrogen can form a pyrrolidine, piperidine or morpholine ring, and then further processing the reaction mixture, characterized in that
- a (cyclo)aliphatic diisocyanate or a corresponding mixture of diisocyanates each comprising 6 to 15 carbon atoms is employed as the diisocyanate,
- the reaction temperature is 0 to 100°C
- the reaction mixture, after reaching a dimerisation degree of 10 to 80%, and in particular 20 to 60%, is subjected to thin film vacuum distillation, using a vacuum of 0.1 to 20 mbar and a temperature of 150-190°C, in order to isolate the urethane dione.

2. A process according to claim 1, characterized in that the diisocyanate used is isophorone diisocyanate.

3. A process according to claim 1 and claim 2, characterized in that the substituted pyridine is added in an amount of 0.05 to 10%, and in particular of 0.2 to 5%, with respect to the weight of the diisocyanate added.

4. A process according to claims 1 to 3, characterized in that the substituents R₁ and R₂ of the pyridine represent methyl or ethyl groups.

5. A process according to claims 1 to 4, characterized in that dimerization is performed at a temperature of 20 to 80°C.

6. A process according to claims 1 to 4, characterized in that dimerization is performed in the presence of a solvent.

7. A process according to claims 1 to 6, characterized in that the distillate produced in the thin film vacuum distillation apparatus, which consists of unconverted diisocyanate and catalyst, is fed back again.

8. A process according to claims 1 to 6, characterized in that dimerization is performed in the presence of a protective gas.

## Revendications

1. Procédé d'obtention d'uretdiones ayant un degré de pureté de plus de 99 %, par réaction des diisocyanates correspondants dans des conditions anhydres, avec des pyridines de formule générale : 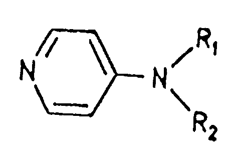 dans laquelle R₁ et R₂ respectivement, indépendamment l'un de l'autre, représentent un radical alcoyle ayant de 1 à 4 atomes de carbone ou ensemble avec l'azote peuvent former un cycle pyrolidine, piperidine ou morpholine
et purification consécutive du mélange réactionnel,
caractérisé en ce que :
- on met en oeuvre comme diisocyanate, un diisocyanate (cyclo)aliphatique ou un mélange correspondant de diisocyanates ayant respectivement de 6 à 15 atomes de carbone,
- la température de réaction se situe de 0 à 100°C et,
- on soumet le mélange réactionnel après avoir atteint un degré de dimérisation de 10 à 80 %, en particulier de 20 à 60 %, à une évaporation en couche mince sous vide, à un vide de 0,1 à 20 mbar et à une température de 150 à 190°C, pour l'isolement de l'uretdione.

2. Procédé selon la revendication 1, caractérisé en ce que l'on met en oeuvre comme diisocyanate, le diisocyanate d'isophorone.

3. Procédé selon les revendications 1 et 2, caractérisé en ce que l'on met en oeuvre la pyridine substituée en une quantité de 0,05 à 10 %, en particulier de 0,2 à 5 %, rapporté au poids de diisocyanate mis en oeuvre.

4. Procédé selon les revendications 1 à 3, caractérisé en ce que les substituants R₁ et R₂ de la pyridine représentent un radical méthyle ou éthyle.

5. Procédé selon les revendications 1 à 4, caractérisé en ce que la dimérisation est effectuée à une température allant de 20 à 80°C.

6. Procédé selon les revendications 1 à 4, caractérisé en ce que l'on effectue la dimérisation en présence de solvants.

7. Procédé selon les revendications 1 à 6, caractérisé en ce que le distillat qui est produit dans l'évaporateur en couche mince sous vide, qui est formé du diisocyanate qui n'a pas réagi et du catalyseur, est recyclé à nouveau.

8. Procédé selon les revendications 1 à 6, caractérisé en ce que l'on effectue la dimérisation en présence d'un gaz protecteur.
